# EUROPEAN PATENT SPECIFICATION

(11) **EP 1 347 302 B1**
(45) Date of publication and mention of the grant of the patent: **21.02.2007**
(21) Application number: 03251762.5
(22) Date of filing: 20.03.2003
(51) Int. Cl.: G01N 33/543, C12Q 1/00

(54) **Direct immunosensor assay**
Direktes Immunosensortestverfahren
Essai immunosenseur direct

(30) Priority: 21.03.2002 US 105050
(43) Date of publication of application: 24.09.2003
(73) Proprietor: LifeScan, Inc., Milpitas, CA 95035 (US)
(72) Inventor: Hodges, Alastair, Blackburn South, Victoria 3130 (AU); Chatelier, Ronald, Bayswater, Victoria 3135 (AU)
(74) Representative: Mercer, Christopher Paul

(56) References cited:
- EP-A- 0 422 708
- EP-A- 0 603 954
- WO-A-02/08763
- US-A1- 2002 012 943
- US-B1- 6 214 205
- US-B1- 6 218 134
- US-B1- 6 325 973

## Description

### Field of the Invention

The present invention relates to a device and method for performing immunoassays. The device comprises a disposable immunosensor.

### Background of the Invention

Biomedical sensors are used to report the presence and/or concentration of a wide variety of analytes. When the analyte is a protein, then the sensing element used is usually an antibody since the interaction of the antibody with the protein (antigen) is very specific. Such immunoassays usually fall into two categories: a "yes/no answer" obtained, e.g., by simple visual detection, or a concentration of the antigen determined by a quantitative method. Most of the quantitative methods involve expensive pieces of equipment such as scintillation counters (for monitoring radioactivity), spectrophotometers, spectrofluorimeters *(see, e.g.,* U.S. 5,156,972), surface plasmon resonance instruments *(see, e.g.,* U.S. 5,965,456), and the like. It would therefore be advantageous to develop a quantitative immunoassay that is both inexpensive and simple enough to use to be suitable for home or field use. Such an immunosensor requires no centrifugation, dilution, pipetting, washing, or timing steps, and generates minimal waste.

Conventional immunoassays are classified into two categories: competition assays and sandwich assays. In a competition assay, the antigen in the test sample is mixed with an antigen-probe complex (commonly referred to as a reporter complex) and the mixture then competes for binding to the antibody. The probe may be a radioisotope, an enzyme, a fluorophore, or a chromophore. In a sandwich immunoassay, the antigen in the test sample binds to the antibody and then a second antibody-probe complex binds to the antigen. In these prior art assay methods, one or more washing steps are usually required. The washing steps introduce complexity into the assay procedure and can generate biohazardous liquid waste. It would therefore be advantageous to develop a device for performing an immunoassay that does not require any washing steps and is suitable for a single use as a disposable device.

### Summary of the Invention

A quantitative, inexpensive, disposable immunosensor that requires no wash steps and thus generates no liquid waste is provided. For immunosensors of certain embodiments, no timing steps are required of the user, and the sensor can be readily adapted to antigen-antibody interactions over a wide kinetic range. The sensors of the preferred embodiments have a number of potential advantages. Such sensors may be simpler to fabricate, as reagents may be deposited in a single step and/or on only one portion of the reaction chamber or a support contained therein.

The sensors may utilize a pseudo-antigen-probe complex, a modified-antigen-probe complex, or an antigen-probe complex. The term "pseudo-antigen," as used herein, is a broad term and is used in its ordinary sense, including, without limitation, antigens other than the antigen of interest that bind to the immobilized antibody, but not as strongly as the antigen of interest. The term "modified-antigen," as used herein, is a broad term and is used in its ordinary sense, including, without limitation, antigens that have been chemically or otherwise modified such that the modified-antigen binds to the immobilized antibody, but not as strongly as the antigen of interest. The antigen of the antigen-probe complex, which may be the same as or different than the antigen of interest, by virtue of being bound to a probe will bind to the immobilized antibody, but not as strongly as the antigen of interest, which is in an unbound state. While the preferred embodiments are discussed primarily in regard to a pseudo-antigen, it is understood that an antigen-probe complex or modified-antigen may be substituted for a pseudo-antigen.

It may be easier to ensure that the ratio of antibody to antigen-probe, modified-antigen-probe, or pseudo-antigen-probe in the reaction chamber is correct as this will essentially occur automatically when the antigen-probe, modified-antigen-probe, or pseudo-antigen-probe is bound to the antibody during manufacture of the sensor, in contrast to prior art methods where the correct ratio is typically achieved by controlling reagent lay-down rates and surface densities. The sensor of preferred embodiments may also be particularly well suited to slower immuno-reaction kinetics, wherein the binding processes may be slow. The use of a non-human pseudo-antigen in the manufacture of the sensor may reduce the likelihood of transmission of communicable diseases when the sensor contacts a drop of blood on the patient's finger.

In a first embodiment, a disposable device for use in detecting a target antigen in a fluid sample is provided, the device including a reaction chamber; an immobilized antibody fixed within the reaction chamber; a reporter complex including a probe and a reporter complex antigen, wherein the probe is linked to the reporter complex antigen, wherein the reporter complex antigen is bound to the immobilized antibody, and wherein the reporter complex antigen binds less strongly than the target antigen to the immobilized antibody; a detection chamber; a sample ingress to the reaction chamber; and a sample passageway between the reaction chamber and the detection chamber.

In an aspect of the first embodiment, the reporter complex antigen may be a target antigen, a pseudo-antigen, or a modified-antigen. The probe may include radioisotopes, chromophores, or fluorophores.

In an aspect of the first embodiment, the probe may include an enzyme, such as glucose dehydrogenase. When the probe is an enzyme, the detection chamber may further include an enzyme substrate, for example, an oxidizable substrate such as glucose. The detection chamber may also further include a mediator, such as dichlorophenolindophenol, or complexes between transition metals and nitrogen-containing heteroatomic species, or ferricyanide. The device may further include a buffer that adjusts the pH of the sample, such as a phosphate or a mellitate. The device may also include a stabilizer, wherein the stabilizer stabilizes one or more of the target antigen, the reporter complex antigen, the enzyme, and the immobilized antibody. The enzyme substrate may be supported on a detection chamber interior surface.

In an aspect of the first embodiment, the immobilized antibody may be supported on a reaction chamber interior surface.

In an aspect of the first embodiment, the device also includes a support material. The support material may be contained within the detection chamber, and may include a first substance such as an enzyme substrate, a mediator, or a buffer, that may be supported on or contained within the support material. The support material may be contained within the reaction chamber, and may include a second substance such as immobilized antibody, the reporter complex, or an agent that prevents non-specific binding of proteins to a reaction chamber internal surface, that may be supported on or contained within the support material. The support material may include a mesh material, for example a mesh material including a polymer such as polyolefin, polyester, nylon, cellulose, polystyrene, polycarbonate, polysulfone, or mixtures thereof. The support material may include a fibrous filling material, such as a fibrous filling material including a polymer such as polyolefin, polyester, nylon, cellulose, polystyrene, polycarbonate, polysulfone, or mixtures thereof. The support material may include a porous material, such as a sintered powder, or a macroporous membrane, for example, a macroporous membrane including polymeric material such as polysulfone, polyvinylidene difluoride, nylon, cellulose acetate, polymethacrylate, polyacrylate, or mixtures thereof. The support material may include a bead.

In an aspect of the first embodiment, the detection chamber includes a first electrode and a second electrode. At least one of the first electrode and the second electrode includes a material such as aluminum, copper, nickel, chromium, steel, stainless steel, palladium, platinum, gold, iridium, carbon, carbon mixed with binder, indium oxide, tin oxide, a conducting polymer, or mixtures thereof.

In an aspect of the first embodiment, a detection chamber wall may be transparent to a radiation emitted or absorbed by the probe, and the radiation is indicative of a presence or absence of the reporter complex in the detection chamber.

In an aspect of the first embodiment, the device includes a detector that detects a condition wherein the reaction chamber is substantially filled.

In an aspect of the first embodiment, the device includes a piercing means that forms a detection chamber vent in a distal end of the detection chamber. The device may also include a reaction chamber vent at a distal end of the reaction chamber.

In an aspect of the first embodiment, the target antigen includes a human C-reactive protein. The reporter complex antigen may include a monomeric C-reactive protein. Alternatively, the reporter complex antigen may include a C-reactive protein derived from a non-human species, or a chemically-modified C-reactive protein, wherein an affinity of the chemically-modified C-reactive protein to the antibody is less than an affinity of the human C-reactive protein to the antibody

In an aspect of the first embodiment, a wall of the detection chamber or a wall of the reaction chamber includes a material such as polyester, polystyrene, polycarbonate, polyolefin, polyethylene terephthalate, or mixtures thereof. The wall of the detection chamber or the wall of the reaction chamber may also include a filler, such as titanium dioxide, carbon, silica, glass, and mixtures thereof.

In an aspect of the first embodiment, the probe includes an enzyme co-factor, such as flavin mononucleotide, flavin adenine dinucleotide, nicotinamide adenine dinucleotide, or pyrroloquinoline quinone. The enzyme co-factor may be linked to the reporter complex antigen through a flexible spacer. The detection chamber may also include an enzyme substrate, or an apoenzyme.

In an aspect of the first embodiment, the probe includes an enzyme activity regulator, such as a kinase or phosphorylase. The detection chamber may also include an enzyme substrate, or an enzyme.

In an aspect of the first embodiment, the probe includes a protein subunit which is part of a multi-subunit enzyme.

In a second embodiment, a method for determining an amount of a target antigen in a fluid sample is provided, the method including the steps of: placing the fluid sample in a reaction chamber containing an immobilized antibody and a reporter complex including a probe linked to a reporter complex antigen, wherein the antibody is fixed within the reaction chamber, wherein the reporter complex antigen is bound to the immobilized antibody, and wherein the reporter complex antigen binds less strongly than the target antigen to the immobilized antibody; dissociating a portion of the reporter complex antigen from the immobilized antibody into the fluid sample; binding a portion of the target antigen to the immobilized antibody; transferring the fluid sample to a detection chamber; and determining an amount of reporter complex in the fluid sample, wherein the amount of reporter complex is indicative of the amount of target antigen initially in the fluid sample.

In an aspect of the second embodiment, the step of transferring the fluid sample to a detection chamber includes transferring the fluid sample to an electrochemical cell having a first electrode and a second electrode. The step of determining an amount of reporter complex in the fluid sample may also include: applying a potential between the first electrode and the second electrode in the electrochemical cell; and measuring a current, wherein the current is indicative of an amount of reporter complex present in the fluid sample, and wherein the amount of reporter complex is indicative of the amount of target antigen.

In an aspect of the second embodiment, the step of transferring the fluid sample to a detection chamber includes transferring the fluid sample to a detection chamber including an electromagnetic radiation transmissive portion. The step of determining an amount of reporter complex in the fluid sample may also include the steps of: exposing the electromagnetic radiation transmissive portion to electromagnetic radiation, whereby the electromagnetic radiation passes through the fluid sample or reflects from the fluid sample; and monitoring a property of the electromagnetic radiation after it passes through the fluid sample or reflects from the fluid sample, wherein the property is indicative of an amount of reporter complex present in the fluid sample, and wherein the amount of reporter complex is indicative of the amount of target antigen.

### Brief Description of the Drawings

FIG. 1 shows a top view (not to scale) of an immunosensor of a first preferred embodiment that incorporates an electrochemical cell.
FIG. 2 shows a cross-sectional view (not to scale) along line A-A' of an embodiment of the immunosensor of Figure 1.
FIG. 3 shows a top view (not to scale) of an immunosensor of a preferred embodiment that incorporates an electrochemical cell.
FIG. 4 shows a cross-sectional view (not to scale) along line B-B' of an embodiment of the immunosensor of Figure 3.

### Detailed Description of the Preferred Embodiments

A sensor strip is provided that contains two chambers: a reaction chamber and a detection chamber. A sample is received in the reaction chamber, wherein components of the sample undergo an immuno-reaction. One or more products of the immuno-reaction are detected in the detection chamber in order to quantitate the antigen present in the sample. The reaction chamber and detection chamber are arranged such that sample may flow from the reaction chamber into the detection chamber.

After the immuno-reaction has taken place in the reaction chamber, at least some of the reacted sample is transferred to the detection chamber, where the presence of a probe is detected and analyzed to obtain a result. It is preferred that sufficient sample is transferred such that the detection chamber is sufficiently filled, namely, that sufficient sample is transferred to the detection chamber such that the presence of a probe may be detected and analyzed by the detection method employed.

The reaction chamber contains antibodies to the antigen of interest immobilized within it. The antibodies can be immobilized on a wall of the chamber itself. Alternatively the antibodies may be immobilized on a support contained within the reaction chamber. Suitable supports include, but are not limited to, fibrous materials, macroporous materials, powdered materials, or, in particularly preferred embodiments, beads of a material such as are commonly known in the art for supporting antibodies.

In the preferred embodiments, the immobilized antibodies are bound to what is referred to as a "pseudo-antigen" linked to a probe. The pseudo-antigen-probe binds to the immobilized antibody, but not as strongly as the antigen of interest. If, for example, the antigen to be detected is a human protein, then a suitable pseudo-antigen-probe may include an animal version of the same protein, such as a dog protein or a pig protein, linked to the probe. In this example, antibodies to the human version of the protein are immobilized in the reaction chamber and the animal version of the protein, linked to a suitable probe, is bound to the immobilized antibody to form an antibody-pseudo-antigen-probe complex.

When sample fills the reaction chamber, a small fraction of the pseudo-antigen-probe dissociates into solution, since it is relatively weakly bound to the antibody. A dynamic equilibrium will exist between bound pseudo-antigen-probe and free pseudo-antigen-probe, leaving some free antibody binding sites. If there is antigen in the solution, then it will strongly bind to the free antibody binding sites in preference to the pseudo-antigen-probe and so leave the pseudo-antigen-probe in solution. This process will continue until substantially all of the antigen in the sample has bound to the antibodies and there is an equal amount of pseudo-antigen-probe free in the solution. Thus each antigen that binds to an immobilized antibody will displace one pseudo-antigen-probe into solution.

When all, or a pre-determined fraction, of the antigen in the sample is bound to the immobilized antibodies, the concentration of pseudo-antigen-probe in solution reflects the original concentration of antigen in the sample. In the preferred embodiments, the equilibrium between free and bound pseudo-antigen-probe is relied upon to ensure that antigen in solution ends up bound to the antibody in preference to the pseudo-antigen-probe. Hence, a pseudo-antigen-probe is employed that binds more weakly to the antibody than the target antigen, but there is no need to physically remove the pseudo-antigen-probe from the antibody prior to sample introduction, as in certain prior art methods.

After the immuno-reactions have taken place, the liquid sample containing any pseudo-antigen-probe liberated from the antibodies is transferred to the detection chamber. In the detection chamber, the concentration of pseudo-antigen-probe present in the sample is measured and a result obtained.

A small amount of the pseudo-antigen-probe may dissociate into solution even in the absence of antigen in the sample, as a result of the bound and free pseudo-antigen-probe reaching equilibrium in solution. If this occurs, then the signal generated in the detection chamber due to this free pseudo-antigen-probe is treated as a background signal, which is subtracted from the antigen concentration result as part of the analysis procedure.

WO02/06788, WO02/06806 WO02/06828 and WO02/08763, an immunoassay strip with a linked immuno-reaction and detection chamber is described. Unlike the sensor described herein, which employs a pseudo-antigen-probe initially complexed with an antibody immobilized on a surface within the reaction chamber, in the sensor of the above-mentioned applications, prior to the introduction of sample into the reaction chamber, antibodies are immobilized on one surface and antigen-probe is immobilized on another surface of the reaction chamber. When sample is introduced into the reaction chamber, the antigen-probe dissolves into the solution and competes with antigen in the sample for the antibody sites. The method of using the sensor of the above-referenced applications, relies primarily on kinetic factors to ensure that the antigen binds to the antibody (by getting there first) in preference to the antigen-probe. Hence, there is a need to spatially remove the antigen-probe from the antibody in the reaction chamber, and the sensor can function when the antigen and the antigen-probe bind with equal strength to the antibody.

In preferred embodiments, the sensor is a single step, no-wash immunosensor. The sensor is a single use, disposable device that employs a reaction chamber and a detection chamber. Any suitable detection method can be utilized. Suitable detection methods include, for example, visual detection wherein the development of a color is observed, or spectroscopic detection wherein reflected or transmitted light is used to measure changes in light absorbance. In a preferred embodiment, the detection method is electrochemical, wherein the electrical current or potential related to the products of immuno-reactions is measured.

Methods and devices for obtaining electrochemical measurements of fluid samples are discussed further in the above-referenced International applications.

The timing of the various test stages, i.e., the reaction stage and the detection stage, may be done manually. Alternatively, timing may be done automatically in response to a trigger signal generated when the reaction chamber and/or detection chamber is filled.

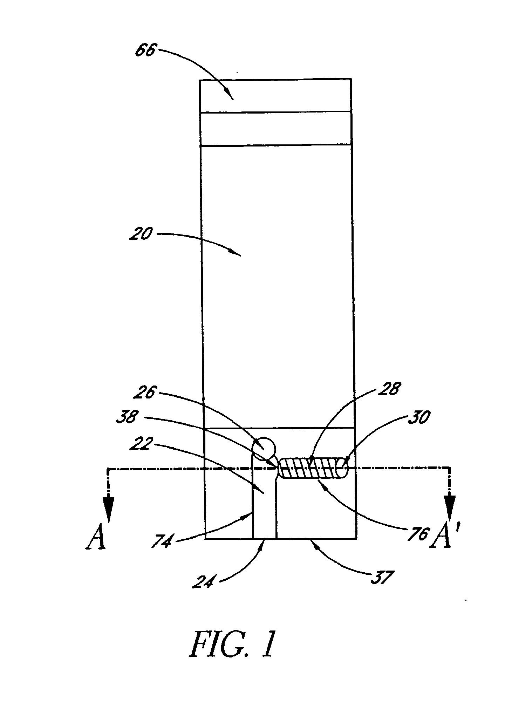

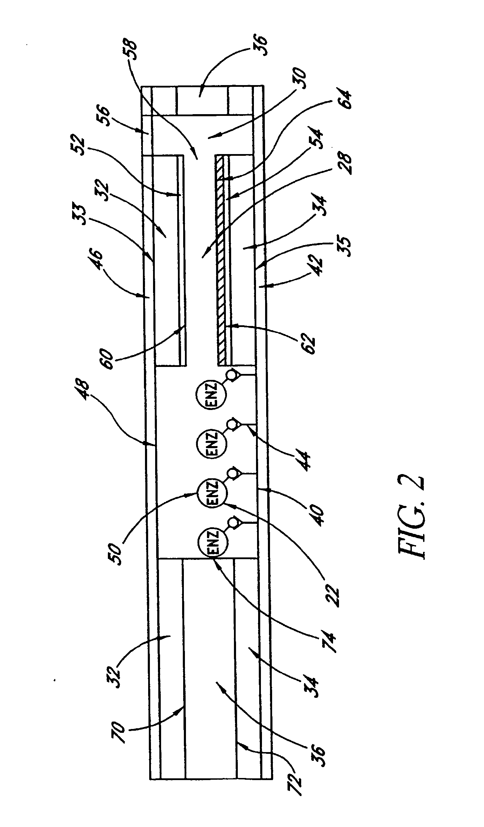

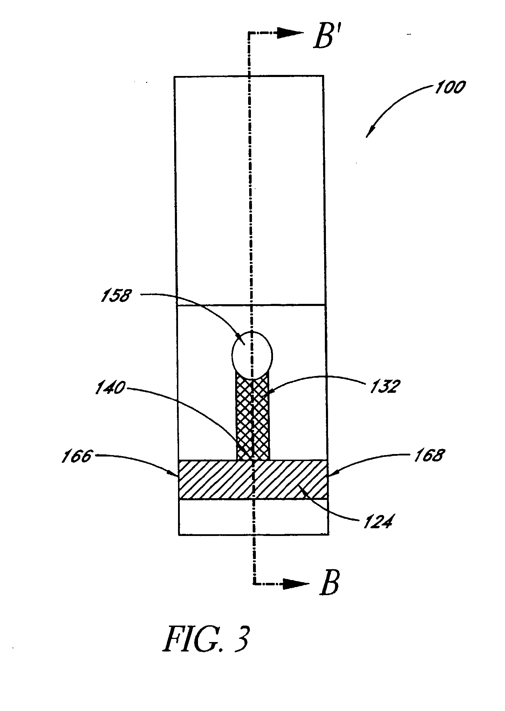

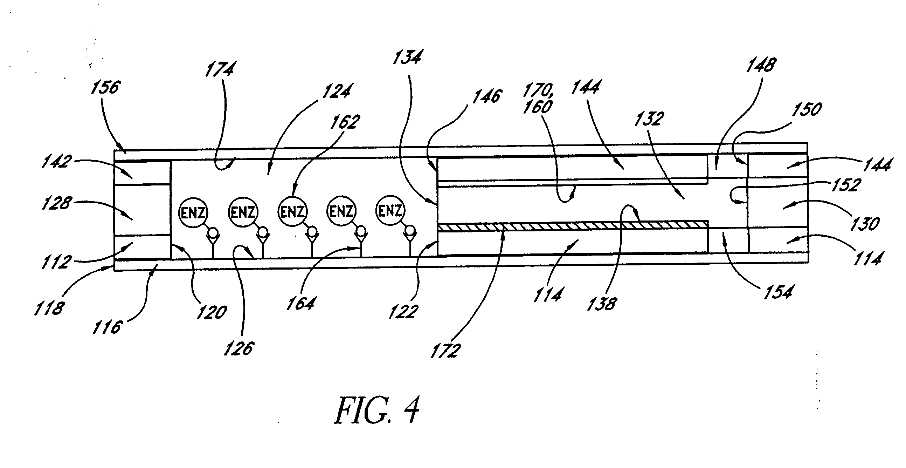

Embodiments of sensors suitable for use with electrochemical detection are illustrated in Figures 1 and 2 and in Figures 3 and 4. Figure 1 is a top view of a first embodiment of a sensor strip and Figure 2 is a cross-sectional view, showing details of the reaction chamber and the detection chamber. Figure 3 is a top view of a second embodiment of a sensor strip and Figure 4 is a cross-sectional view, showing details of the reaction chamber and the detection chamber.

### The Sensor

The immunosensors of the present invention may be prepared using well-known thin layer device fabrication techniques as are used in preparing electrochemical glucose sensing devices (see, e.g., U.S. 5,942,102). Such techniques, with certain modifications, may also used to prepare immunosensors utilizing non-electrochemical detection methods.

In the preferred embodiments of the immunosensors illustrated in Figures 1 and 2 and in Figures 3 and 4, the detection chamber comprises an electrochemical cell. The immunosensors may be prepared by assembling various thin layers of suitably shaped materials according to thin layer sensor fabrication methods as are well known in the art. Typically, the fabrication process involves sandwiching one or more spacer layers between a top layer and a bottom layer.

In a preferred embodiment, the sensor **20** is an electrochemical cell **28** utilizing an enzyme, e.g., glucose oxidase or glucose dehydrogenase, as the probe, as illustrated in Figure 1, a top view of such a sensor **20**, and Figure 2, a cross section of the sensor through line A-A'. The reaction chamber **22** and detection chamber **28** are prepared by forming an aperture extending through a sheet of electrically resistive material **36**. The aperture is shaped such that it defines a sidewall of both the reaction chamber **22** and the detection chamber **28**, as well as the sample passageway **38** between the two chambers **22** and **28**. By extending the aperture from the proximal end **24** of the reaction chamber **2**2 through to the edge of the sheet **37,** the sample ingress **24** is also formed. In one embodiment, the thickness of the sheet **36** defines the entire height of the reaction chamber **22** and detection chamber **28**, which are the same. In another embodiment, the height of the reaction chamber **22** is greater than that of the detection chamber **28**. A reaction chamber **22** of greater height than the detection chamber **28** is prepared by layering multiple sheets **32**, **34**, and **36** together. The middle sheet **36** of the layer has an aperture defining the sidewalls **74** and **76** of both the reaction chamber **22** and detection chamber **28** as described above. This middle layer **36** is sandwiched between two or more additional layers **32** and **34**, the additional layers **32** and **34** having an aperture defining the side wall **74** of the reaction chamber **22** only, the laye'rs **32** and **34** thereby defining end walls **60** and **62** of the detection chamber **28**. In this embodiment, the end walls **60** and **62** of the detection chamber comprise electrodes **52** and **54**, which may be prepared as described below.

As illustrated in Figure 2, antibodies **44** are tethered to the bottom **40** of the reaction chamber **22**. The pseudo-antigen-probe **50** is complexed to the antibodies **44**. The antibody may be tethered to any suitable surface within the reaction chamber, e.g. tethered to a wall or on a surface of a support within the reaction chamber **22**.

A first thin electrode layer **52** is mounted or formed on one side **70** of the sheet of electrically resistive material **36**, extending over the aperture forming the detection chamber **28** and forming an end wall **60**. The layer **52** may be adhered to the sheet **36**, e.g., by an adhesive. Suitable adhesives include, for example, heat activated adhesives, pressure sensitive adhesives, heat cured adhesives, chemically cured adhesives, hot melt adhesives, hot flow adhesives, and the like.

The electrode layer **52** may be prepared by coating (e.g., by sputter coating as disclosed in WO97/18464, by screen printing, or by any other suitable method) a sheet of electrically resistive material **32** with a suitable material, for example, aluminum, copper, nickel, chromium, steel, stainless steel, platinum, palladium, carbon, carbon mixed with a binder, indium oxide, tin oxide, mixed indium/tin oxides, gold, silver, iridium, mixtures thereof, conducting polymers such as polypyrrole or polyacetylene, and the like. If electrode **52** is to be used as a cathode in the electrochemical cell, then suitable materials include, for example, aluminum, copper, nickel, chromium, steel, stainless steel, platinum, palladium, carbon, carbon mixed with a binder, indium oxide, tin oxide, mixed indium/tin oxides, gold, silver, iridium, mixtures thereof, conducting polymers such as polypyrrole or polyacetylene, and the like. If electrode **52** is to be used as an anode in the electrochemical cell or is to come into contact with oxidizing substances during sensor manufacture or storage, then suitable materials include, for example, platinum, palladium, carbon, carbon mixed with a binder, indium oxide, tin oxide, mixed indium/tin oxides, gold, silver, iridium, mixtures thereof, conducting polymers such as polypyrrole or polyacetylene, and the like. Materials suitable for use as electrodes **52** and **54** are compatible with the reagents present in the sensor **20**, namely, they do not react chemically with reagents at the potential of choice or during sensor fabrication and storage.

A second thin electrode layer **54** is mounted on the opposite side **72** of the electrically resistive material **36**, also extending over the aperture forming the detection chamber **28**, so as to form a second end wall **62**. In this embodiment, the inert, electrically insulating layer **36** separates the electrode-bearing substrates **32** and **34**. Preferably, insulating layer **36** keeps layers **32** and **34** at a predetermined separation. Provided this separation is small enough, e.g., less than or equal to about 500 microns, the current flowing between the electrodes **52** and **54** will be directly proportional to the concentration of reduced mediator after a suitably short time relative to the detection time employed. In this embodiment, the rate of current rise is directly related to the rate of the enzyme reaction and therefore the amount of enzyme present.

In certain embodiments, an electrode configuration other than an opposing relationship may be preferred, for example, a side-by-side relationship, or a parallel but offset relationship. The electrodes may be identical or substantially similar in size, or may be of different sizes and/or different shapes. The electrodes may comprise the same conductive material, or different materials. Other variations in electrode configuration, spacing, and construction or fabrication will be apparent to those of skill in the art.

In a preferred embodiment, the electrode layers **52** and **54** are mounted in a parallel opposing relationship at a distance of less than or equal to 500, 450, 400, 350, 300, 250, or 200 microns, and more preferably from about 5, 10, 15, 20, 25, 30, 35, 40, 45, or 50 microns to about 75, 100, 125, 150, or 175 microns. In certain embodiments, however, it may be preferred that the electrode spacing is greater than 500 microns, for example, 600, 700, 800, 900, or 1000 microns, or even greater than 1, 2, 3, 4, or 5 millimeters.

The volume of the detection chamber or the reaction chamber is typically about 0.3 microliters or less to about 100 microliters or more, preferably about 0.5, 0.6, 0.7, 0.8, or 0.9 microliters to about 20, 30, 40, 50, 60, 70, 80, or 90 microliters, and most preferably about 1.5, 2, 2.5, 3, 3.5, 4, 4.5, or 5 microliters to about 6, 7, 8, 9, 10, 12, 14, 16, or 18 microliters. However, in certain embodiments, larger or smaller volumes may be preferred for one or both of the reaction chamber and the detection chamber.

The electrodes **54** and **52** in the detection chamber **28** can be placed in electrical connection with a meter (not shown) through the connection end **66**. The connectors (not shown) are in electrical connection with the electrodes **54** and **56** in the detection chamber **28** via conducting tracks (not shown). In the preferred embodiment illustrated in Figure 1, these conducting tracks consist of extensions of the films of conductor **52** and **54** coated onto the internal surfaces of **32** and **34**. The meter in connection with the connection area **66** is capable of applying a potential between the electrodes **52** and **54** in the detection chamber **28**, analyzing the electrical signals generated, displaying a response, optionally storing the response in memory, and optionally transmitting stored responses to an external device such as a printer or computer.

In other embodiments utilizing electrochemical detection, stripes of conducting material on one or both internal faces of the detection chamber are typically used, with at least two electrodes present, namely, a sensing electrode and a counter/reference electrode. Optionally, a third electrode, serving as a separate reference electrode, may be present.

When utilizing potentiometric detection methods, the meter is capable of measuring the potential difference between a sensing electrode and a reference electrode, but need not be capable of applying a potential between the electrodes.

In embodiments wherein visual detection or reflectance spectroscopy is the detection method used, the layers **32** and **46** and/or layers **34** and **42** are transparent to the wavelength of radiation that is to be observed. In the case of visual detection, a simple color change in the detection chamber **28** is observed. In the case of reflectance spectroscopy, detection radiation is shone through layers **32** and **46** and/or layers **34** and **42,** and radiation reflected from the solution in the detection chamber **28** is analyzed. In the case of transmission spectroscopy as the detection method, layers **32**, **46**, **34**, and **42** are transparent to radiation at the wavelength of choice. Radiation is shone through the sample in the detection chamber **28** and the attenuation of the beam is measured.

In a preferred embodiment, layer **36** comprises a substrate with a layer of adhesive (not shown) coated on its upper surface **70** and lower surface **72**. Examples of materials suitable for the substrate of layer **36** include polyester, polystyrene, polycarbonate, polyolefins, and, preferably, polyethylene terephthalate. These may be native materials or may be filled with suitable fillers to confer desirable optical or mechanical properties. Examples of materials suitable as fillers include, but are not limited to, titanium dioxide, carbon, silica, and glass. Examples of suitable adhesives are pressure sensitive adhesives, heat and chemically curing adhesives and hot melt and hot flow adhesives. Alternatively, the spacer layers themselves may consist of a suitable adhesive.

If a sample ingress **24** has not already been formed earlier in the fabrication process, then one is provided, for example, by forming a notch (not illustrated) in the edge **37** of the device **20** that intersects the reaction chamber **22**.

The dashed circle in Figure 1 denotes an aperture **30** piercing layers **32**, **34**, and **36** but not layers **42** and **46**, the aperture in layer **34** opening into the detection chamber **28**. Since layers **42** and **46** are not pierced initially, the only opening to the atmosphere of the detection chamber **28** is the sample passageway 38 opening into the reaction chamber **22**. Thus, when the reaction chamber **22** fills with sample, the sample passageway **38** to the detection chamber **28** is blocked. This traps air in the detection chamber **28** and substantially prevents it from filling with sample. A small amount of sample will enter the detection chamber **28** during the time between when the sample first contacts the opening **38** to the detection chamber **28** and when the sample contacts the far side of the opening **38**. However, once the sample has wet totally across the opening **38** to the detection chamber **28**, no more filling of the detection chamber **28** will take place. The volume of the reaction chamber **22** is typically chosen so as to be at least equal to and preferably larger than the volume of the detection chamber **28**. By opening the vent **30** to the atmosphere, sample is transferred to fill the detection chamber **28**. The vent may be opened by means of a needle connected to a solenoid in the meter.

An immunosensor **100** of another embodiment, as depicted in Figures 3 and 4, may be prepared as follows. A first shaped layer **112** and a second shaped spacer **114** layer of similar thickness are each situated atop a bottom layer **116**. The first spacer layer **112** is rectangular in shape, and is situated at the proximal edge **118** of the bottom layer **116**. The second spacer layer **114** is also rectangular in shape, and is situated on the bottom layer **116** at a distance apart from the first spacer layer **112**. The distal edge 120 of the first spacer layer **116** and the proximal edge **122** of the second spacer layer **114** form portions **120**, **122** of the side walls of the reaction chamber **124**. The bottom layer **116** forms the bottom wall **126** of the reaction chamber **124**. Antibodies **164** are tethered to the bottom **126** of the reaction chamber **124**. The antigen-probe or pseudo-antigen-probe **162** is bound to the tethered antibodies **164**.

A third shaped spacer layer **128**, similar in shape to the first shaped spacer layer **112**, is situated atop the first shaped spacer layer **112**. A fourth spacer layer **130** has a slit **132** extending through the proximal end **134** of the spacer layer **130** towards the center of the spacer layer **130**. The fourth spacer layer is **130** situated atop the second shaped spacer layer **114** with the proximal ends **122**, **134** aligned. The slit **132** in the fourth spacer layer forms the sidewalls (not illustrated) of the detection chamber **132**. The portion **138** of the second spacer layer exposed by the slit **132** in the fourth spacer layer **130** forms the bottom **138** of the detection chamber **132**. The proximal end **140** of the slit **132** forms the passageway **140** between the reaction chamber **124** and the detection chamber **132**. The proximal end **134** of the fourth spacer layer **130** forms a portion **134** of the sidewall of the reaction chamber **124**.

A fifth shaped spacer **142**, similar in shape to the first shaped spacer layer **112** and third shaped spacer layer **128**, is situated atop the third spacer layer **128**. A sixth shaped spacer layer **144**, similar in shape to the second shaped spacer layer **114**, is placed atop the fourth shaped spacer layer **130**, with the proximal ends **146**, **122** aligned. The portion **170** of the sixth spacer layer exposed by the slit **132** in the fourth spacer layer **130** forms the top **170** of the detection chamber **132**. An aperture **148** extends through the sixth shaped spacer layer **144**. The distal end **150** of the aperture **148** and the distal end **152** of the slit **132** are aligned. The aperture **148** forms a portion **150** of a sidewall of a vent **154**, allowing displacement of air from the detection chamber **132** as it fills with sample. A top layer **156** is fitted over the fifth spacer layer **142** and sixth spacer layer **144**. The top layer **156** also includes an aperture **158** of similar size and shape and in alignment with the aperture **148** in the sixth shaped layer **144**.

In certain embodiments, it may be preferred to delay the filling of the detection chamber **132** to some time after sample has filled the reaction chamber **124**, to allow time for the immuno-reactions to proceed in the reaction chamber **124**. In these embodiments, this is achieved by forming a vent hole **158** in layer **116** and/or **156** after completion of the immuno-reactions. When the reaction chamber **124** fills with sample, air is trapped in the detection chamber **132**, which prevents it from being filled with sample. At a suitable time after sample has filled the reaction chamber **124**, at least one of the top layer **156** and the bottom layer **116** can be punctured above the vent hole **148** or below the vent hole **154** by a suitable device, such as a needle or blade. When this occurs, the air in the detection chamber **132** can vent through the hole **148** or hole **154** formed in layer **116** and/or **156** via aperture **148** or **154**, thus allowing sample to be drawn into the detection chamber **132** from the reaction chamber **124** by capillary action and the displaced air to be vented.

The height of the detection chamber **132** is typically selected to be less than the height of the reaction chamber **124** such that, in combination with the surface energies of the faces of chambers **132** and **124**, the capillary force in the detection chamber **132** will be greater than that in the reaction chamber **124**. The stronger capillary force in the detection chamber **132** serves to draw sample into the detection chamber **132** while emptying the reaction chamber **124**. This method of using differentials in capillary force to fill a chamber is described in detail in copending Application No. 09/536,234 filed on March 27, 2000.

In preferred embodiments, the height of the reaction chamber is typically greater than the height of the detection chamber. The height of the detection chamber is typically about 500 microns or less, preferably about 450, 400, 350, 300, 250 microns or less, and more preferably about 5, 10, 15, 20, 25, 30, 35, 40, 45, or 50 microns to about 75, 100, 125, 150, 175, or 200 microns. These detection chamber heights are particularly well suited to applications wherein the top and bottom walls of the detection chamber comprise electrode layers. However, there may be certain embodiments wherein electrochemical detection is employed wherein cell heights greater than about 500 microns may be preferred. These detection chamber heights may also be suitable when detection methods other than electrochemical detection are employed. When another detection method is employed, for example, an optical detection method, different cell heights may be preferred. In such embodiments, a cell height of about 600, 700, 800, or 900 microns or more, or even about 1, 1.5, 2, 2.5, 3, 3.5, 4, 4.5, or 5 mm or more may be preferred. The height of the reaction chamber is typically greater than that of the detection chamber. However, in certain embodiments it may be preferred to employ a reaction chamber having the same or a similar height as the detection chamber, or even a smaller height than the detection chamber. The detection chamber height is typically from about 5 microns or less to about 1, 1.5, 2, 2.5, 3, 3.5, 4, 4.5, or 5 mm or more, preferably about 900, 800, 700, 600, or 500 microns or less, more preferably about 450, 400, 350, 300, or 250 microns or less, and most preferably from about 5, 10, 15, 20, 25, 30, 35, 40, 45, or 50 microns to about 75, 100, 125, 150, 175, 200, or 250 microns.

When the immunosensor **100** is an electrochemical sensor **100**, the top surface of the second spacer layer **138** and the bottom surface **160** of the sixth spacer layer **144** which are exposed by the slit **132** in the fourth spacer layer **130** may be partially or completely coated with a conducting material. Alternatively, layers **114** and **144** may themselves be made of electrically conductive materials. Electrical connection between the two conducting layers (not illustrated) and a meter (not illustrated) enable electrochemical measurements to be conducted within the detection chamber.

### Fabrication Methods

For purposes of illustration, details of the fabrication of sensors of preferred embodiments are discussed with reference to the sensor depicted in Figures 3 and 4. The sensor strip **100** is typically constructed of layers of material laminated together. One or more spacer layers **128**, **130** are used to space layers **112** and **114** apart from layers **142** and **144.** The spacer layers have adhesive faces to allow layers **112, 128,** and **142** and layers **114, 130**, and **144** to be held together. Alternatively, the spacer layers themselves may consist of an adhesive, or may comprise a material capable of adhering to adjacent layers by the application of heat and/or pressure in a lamination method.

The detection chamber **132** is a capillary space where layers **114** and **144** form the end walls of the space and the thickness of layers **128**, **130** define the height. Layers **114** and **144** can also serve as substrates for electrode coatings (not illustrated) that form the electrodes of an electrochemical cell or may act as the electrodes themselves by virtue of being constructed of electrically conductive materials. In construction, detection chamber **132** is typically formed by punching out, or otherwise removing a portion of layer **130**. This cutout portion of layer **130** can also serve to define the electrode area of the electrochemical cell.

The reaction chamber **124** can be formed by punching or otherwise removing a portion of the spacer layers, with the areas removed such that the reaction chamber overlaps with the detection chamber **132**, thus causing the detection chamber **132** to open into the reaction chamber **124.** Layers **116** and **156** can then be laminated to the external face of layers **112, 114** and layers **142, 144,** respectively, to form the end walls **126, 174** of reaction chamber **124.** The immuno-chemicals **164** and **162** can be coated onto an internal face **126** and/or **174** of layers **116** and/or **156** prior to or following the lamination of **116** and **156** onto layers **112, 114** and layers **142, 144,** respectively. Layers **116** and **156** can be adhered to layers **112**, **114** and layers **142**, **144**, respectively, by an adhesive layer on the external face of layers **112**, **114** and layers **142**, **144**, respectively, or on the internal face of layers **116** and **156**.

The vent **148** and/or **154** can advantageously be formed by punching a hole through layers **114, 130**, and **144**. From the point of view of simplifying the strip fabrication process, it is particularly advantageous to form vent **148** and/or **154** at the same time as the cut-out portion for the reaction chamber **124** and/or the detection chamber **132** is formed, as this makes it easier to achieve a reproducible spatial relationship between the chamber(s) and the vent, and also reduces the number of process steps.

In a different embodiment, the vent **148, 154, 158,** can be formed by punching through layers **114, 116, 130, 144,** and **156** and additional tape layers (not illustrated) laminated over both ends of the hole thus formed. This has the advantage of permitting optimization of the properties of layers **116** and **156** and the vent hole covering tape layers (not illustrated) separately. Alternatively, vent hole **148, 154, 158** can be formed by punching through layers **114, 130, 144** and **116** or **156** prior to the lamination of layers **116** or **156,** respectively. This leaves an opening of **158** to only one face of the strip **100** and thus only one covering tape is used.

In a further embodiment, the layers **116** and **156** can be formed and laminated to layers **114** and **144** such that layers **116** and **156** do not extend to cover the area where the vent **158** is formed. Then it is only necessary to punch through layers **114**, **130** and **144** to form the vent **148**, **154**, **158** and additional tape layers (not illustrated) laminated over both ends of the hole thus formed.

The layers may be adhered to each other by any suitable method, for example, pressure sensitive adhesive, curable adhesives, hot melt adhesives, lamination by application of heat and/or pressure, mechanical fasteners, and the like.

The above-described configurations for the sensor are but two of many possible configurations for the sensor, as will be appreciated by one of skill in the art. For example, the vent may be provided through the top of the strip, the bottom of the strip, both the top and bottom of the strip, or through one or more sides of the strip. The vent may be of any suitable configuration, and may extend directly into a portion of the detection chamber, or may follow a circuitous path into the detection chamber. The detection chamber may be of any suitable shape, for example, rectangular, square, circular, or irregular. The detection chamber may abut the reaction chamber, or a separate sample passageway between the reaction chamber and the detection chamber may be provided. Sample may be admitted to the reaction chamber on either side of the strip, as in the sensor of Figures 4 and 3, or only through one side of the strip with the opposite side blocked by a spacer, as in Figures 1 and 2. The detection chamber may be of any suitable shape, for example, rectangular, square, circular, or irregular. The detection chamber may be contained within the body of the strip, and access to the detection chamber may be provided by one or more sample ingresses through the top, bottom, or sides of the strip. Typically, a particular configuration is selected such that the fabrication method may be simplified, e.g., by performing fewer steps or by using fewer components.

### Electrochemical Detection

When the sensor is an electrochemical cell, the electrode layers, for example, layers **52** and **54** of the sensor of Figures 1 and 2, are provided with an electrical connector allowing the sensor **20** to be placed in a measuring circuit. At least one of the electrodes **52** or **54** in the cell **28** is a sensing electrode, i.e., an electrode sensitive to the amount of oxidized or reduced form of an analyte in the sample. In the case of a potentiometric sensor **20** wherein the potential of the sensing electrode **52** or **54** is indicative of the level of analyte present, a second electrode **54** or **52**, acting as reference electrode is present which acts to provide a reference potential. In the case of an amperometric sensor **20** wherein the sensing electrode current is indicative of the level of analyte in the sample, at least one other electrode **54** or **52** is present which functions as a counter electrode to complete the electrical circuit. This second electrode **54** or **52** may also function as a reference electrode. Alternatively, a separate electrode (not shown) may perform the function of a reference electrode.

If the immunosensor **20** is operated as an electrochemical cell **28,** then the sheet **36** containing the apertures defining the reaction chamber **22** and/or detection chamber **28** comprises an electrically resistive material. In a preferred embodiment, sheets **32** and **34** also comprise an electrically resistive material. Suitable electrically resistive materials include, for example, polyesters, polystyrenes, polycarbonates, polyolefins, mixtures thereof, and the like. Preferred polyester is polyethylene terephthalate. In the sensor depicted in Figures 1 and 2, the layers **32** and **34** are substrates coated with electrically conductive material **52** and **54.** The electrically conductive material **52** or **54** is coated on the surface **60** or **62** facing the detection chamber **28** and an adhesive layer (not shown) is coated on the surface **33** or 35 facing layer **42** or **46**, respectively.

In the embodiment depicted in Figures 3 and 4, the detection chamber **132** has electrically conductive coatings (not illustrated) on the internal face of **138** and **170** which are suitable for use as electrodes in an electrochemical sensor cell. Also contained in the detection chamber **132** is a dry reagent layer **172** comprising a substrate for the probe enzyme and, if necessary, a redox species capable of cycling the enzyme between its oxidized and reduced forms and capable of being oxidized or reduced at the cell electrodes. A buffer may also be present to control pH in the detection chamber **132**. When the immunosensor is in use, the electrodes are connected to an external electronic meter device (not illustrated) through external connectors (not illustrated), for example, tongue plugs, as are known in the art. Suitable connectors are disclosed in copending Application No. 09/399,512 filed on September 20, 1999 and copending Application No. 60/345,743 filed on January 4, 2002.

If the immunosensor **20, 100** is operated using a detection method other than an electrochemical detection method, then the materials from which the sensor is constructed need not be electrically resistive. However, the polymeric materials described above are preferred for use in constructing the immunosensors of preferred embodiments because of their ease of processing, low cost, and lack of reactivity to reagents and samples.

### Optical Detection

In an alternative embodiment, an optical rather than an electrochemical detection system are used. According to this alternative embodiment, electrodes are not necessary and an external light source and photocell are used to analyze light transmitted through, or reflected from the solution in detection chamber. In one embodiment, it is preferred to shine the light through the top surface of the sensor then through the sample, where it is reflected off the lower sensor layer and then back up through the sample and the top layer, where it is detected. In another embodiment, light is shone in through the side of the detection chamber and totally internally reflected between the end faces of the detection chamber until it passes out through the other side of the detection chamber, where it is detected. In these embodiments, the layers above, to the side, and/or below the detection chamber are substantially transparent to the analyzing light that is passed through the layer or layers. The techniques described in copending Application No. 09/404,119 filed on September 23, 1999 may be adapted for use with the immunosensors of preferred embodiments utilizing optical detection systems. Alternatively, in certain embodiments it may be preferred to use a combination of electrochemical detection and optical detection methods, which is also described in Application No. 09/404,119.

### Reagents and Other Materials Present in the Immunosensor

Reagents for use in the reaction chamber, e.g., immobilized antibody, pseudo-antigen-probe, buffer, mediator, and the like, may be supported on the walls of the reaction chamber or on the walls of the detection chamber, on an independent support contained within chambers, within a matrix, or may be self supporting. If the reagents are to be supported on the chamber walls or electrodes, the chemicals may be applied by use of printing techniques well known in the art, e.g., ink jet printing, screen printing, slot coating, lithography, and the like. In a preferred embodiment, a solution containing the reagent is applied to a surface within a chamber and allowed to dry.

Rather than immobilize or dry the reagents or other chemicals onto the surfaces of the reaction chamber or detection chamber, it may be advantageous to support them on or contain them within one or more independent supports, which are then placed into a chamber. Suitable independent supports include, but are not limited to, mesh materials, nonwoven sheet materials, fibrous filling materials, macroporous membranes, sintered powders, gels, or beads. The advantages of independent supports include an increased surface area, thus allowing more antibody and pseudo-antigen-probe to be included in the reaction chamber, if desired. In such an embodiment, the antibody bound to the pseudo-antigen-probe is dried onto a piece of porous material, which is then placed in the reaction chamber. It is also easier during fabrication to wash unbound protein from independent supports, such as beads, compared to washing unbound protein off of the surface of the reaction chamber.

In a particularly preferred embodiment, the antibody bound to the pseudo-antigen-probe is supported on beads. Such beads may comprise a polymeric material, e.g., latex or agarose, optionally encasing a magnetic material (such as gamma Fe₂O₃ and Fe₃O₄). The bead material is selected such that suitable support for the antibody is provided. Suitable beads may include those marketed as DYNABEADS® by Dynal Biotech of Oslo, Norway. Optionally, a magnet may be included in the meter to hold the magnetic beads in the reaction chamber and to stop them from moving to the detection chamber.

In yet another embodiment, the walls of the reaction chamber are porous, with the antibody bound to the pseudo-antigen-probe incorporated into the pores. In this embodiment, the liquid sample is able to wick into the porous wall, but not leak out of the defined area. This is accomplished by using a macroporous membrane to form the reaction chamber wall and compressing the membrane around the reaction chamber to prevent leakage of sample out of the desired area, as described in U.S. Pat. No. 5,980,709 to Hodges, et al.

Suitable independent supports such as beads, mesh materials, nonwoven sheet materials, and fibrous fill materials include, polyolefins, polyesters, nylons, cellulose, polystyrenes, polycarbonates, polysulfones, mixtures thereof, and the like. Suitable macroporous membranes may be prepared from polymeric materials including polysulfones, polyvinylidene difluorides, nylons, cellulose acetates, polymethacrylates, polyacrylates, mixtures thereof, and the like.

The antibody bound to the pseudo-antigen-probe may be contained within a matrix, e.g., polyvinyl acetate. By varying the solubility characteristics of the matrix in the sample, controlled release of the protein or antibody into the sample may be achieved.

As illustrated in Figure 2, dried reagents **64** may optionally be disposed in the detection chamber **28**. These reagents may include an enzyme substrate (used as a probe) and a mediator, capable of reacting with the enzyme part of the pseudo-antigen-enzyme probe **50** to produce a detectable signal. The enzyme substrate and mediator, if present, are to be of sufficient amount such that the rate of reaction of any enzyme present with the enzyme substrate **64** is determined by the amount of enzyme present. For instance, if the enzyme is glucose oxidase or glucose dehydrogenase, a suitable enzyme mediator **64** and glucose (if not already present in the sample) is disposed into the detection chamber **28**.

In an embodiment wherein an electrochemical detection system is used, ferricyanide is a suitable mediator. Other suitable mediators include dichlorophenolindophenol and complexes between transition metals and nitrogen-containing heteroatomic species. Buffer may also be included to adjust the pH of the sample in the detection chamber **28**, if necessary. The glucose, mediator, and buffer reagents **64** are present in sufficient quantities such that the rate of reaction of the enzyme with the enzyme substrate **64** is limited by the concentration of the enzyme present.

The internal surface **40** of the substrate **42**, which forms the base of the reaction chamber **22**, is coated with pseudo-antigen-probe **50** bound to antibodies **44** to the antigen to be detected in the sample. The antibodies **44** are adsorbed or otherwise immobilized on the surface **40** of the substrate **42** such that they are not removed from the substrate **42** during a test. Optionally, during or after application of the antibodies **44** to the internal surface **40** of the substrate **42**, an agent designed to prevent non-specific binding of proteins to this surface can be applied (not shown). An example of such an agent well known in the art is bovine serum albumin (BSA). A nonionic surfactant may also be used as such an agent, e.g., TRITON® X100 surfactant manufactured by Rohm & Haas of Philadelphia, Pennsylvania, or TWEEN® surfactants manufactured by ICI Americas of Wilmington, Delaware. The nonionic surfactant selected does not denature proteins. The coating **44** on the internal surface **40** of the substrate **42** is in the dry state when ready to be used in a test.

In preferred embodiments wherein electrochemical detection is employed, enzymes may be used as the probe. Examples of suitable enzymes include, but are not limited to, horseradish peroxidase, glucose oxidase, and glucose dehydrogenase, for example, PQQ dependent glucose dehydrogenase or NAD dependent glucose dehydrogenase.

The probe can also be an enzyme co-factor. Examples of suitable co-factors include, but are not limited to, flavin mononucleotide, flavin adenine dinucleotide, nicotinamide adenine dinucleotide, and pyrroloquinoline quinone. The co-factor is preferably linked to the antigen by a flexible spacer to allow the co-factor to bind to the apoenzyme. When the probe is a co-factor, the apoenzyme may optionally be co-dried with the enzyme substrate and mediator in the reaction chamber.

The probe can also be a regulator of enzyme activity. Examples of suitable enzyme regulators include, but are not limited to, kinases or phosphorylases. Enzyme regulators may alter the activity of the enzyme by changing the state of phosphorylation, methylation, adenylation, uridylation or adenosine diphosphate ribosylation of the enzyme. Enzyme regulators may also alter the activity of the enzyme by cleaving a peptide off the enzyme. When the probe is an enzyme regulator, the enzyme is co-dried with the enzyme substrate and mediator in the reaction chamber.

The probe can be a protein subunit which is part of a multi-subunit complex. An example of such a protein subunit is one of the subunits in the multi-subunit enzyme cytochrome oxidase.

The antibody and pseudo-antigen-probe can be complexed together before being dried into the reaction chamber. Complexation conditions are chosen to minimize the amount of free (uncomplexed) pseudo-antigen-probe, as this species will increase the background signal in the assay. The amount of free antibody is also minimized as this species will bind antigen and stop it from displacing the pseudo-antigen-probe, thus reducing the sensitivity of the assay. For example, it is possible to optimize the complexation of pseudo-antigen-probes with antibodies by "crowding" the solutions with inert macromolecules, such as polyethylene glycol, which excludes volume to the proteins and thus raises their thermodynamic activity and enhances the affinity of their binding to one another. *See,* e.g., Minton, *Biopolymers*, Vol. 20, pp 2093-2120 (1981).

It is advantageous to have the antibody immobilized on beads before it is complexed to the pseudo-antigen-probe. This allows all the antibody sites to be occupied by exposing them to a high concentration of the pseudo-antigen-probe. Excess pseudo-antigen-probe is then readily removed by centrifugation and washing of the beads.

The immunosensor is most sensitive to antigen concentrations from about 1 nM to about 10 µM (micromolar). For an antigen with a relative molar mass of 100,000, this corresponds to about 0.1 µg/mL (micrograms/mL) to about 1000 µg/mL (micrograms/mL). However, the sensor can be modified (e.g., by changing the separation between the electrodes, or by applying a different pattern of voltage pulses) to assay antigen concentrations in the range 0.1 nM or less to 0.1 mM or more.

The maximum detectable limit of the assay is determined by the concentration of pseudo-antigen-probe/antibody in the reaction chamber. This molar concentration is therefore set to correspond to the expected range of molar antigen concentrations that are typically encountered in samples of interest. For example, the concentration of C-reactive protein encountered in a typical pathology laboratory is from about 10 nM to about 10 µM (micromolar).

Examples of antigens that may be assayed include, but are not limited to, Alpha-fetoprotein, Carcinoembryonic antigen, C-reactive protein, cardiac Troponin I, cardiac Troponin T, Digoxin, ferritin, Gamma glutamyl transferase, Glycated hemoglobin, glycated protein, Hepatitis A, B and C, chorionic gonadotropin, Human immunodeficiency virus, insulin, serum amyloid A, thromblastin, Prostate specific antigen, Prothrombin, Thyroxine, Tumor antigen CA125, Tumor antigen CA15-3, Tumor antigen CA27/29, Tumor antigen CA19-9, and Tumor antigen NMP22.

The sensors of preferred embodiments are not limited to the assay of human antigens, but are also suitable for use in veterinary and animal husbandry applications. Also, if an antigen is too small to be immunogenic, then it can be attached to a carrier as a hapten and antibodies can be raised to it in this way. Therefore the invention is not limited to the assay of protein antigens or to large molecules, but is also applicable to small antigens as well.

Antibodies suitable for use in the sensors of preferred embodiments include, but are not limited to, the natural antibodies, such as IgG, IgM and IgA. Suitable antibodies can also be made up of fragments of natural antibodies, such as F(ab)₂ or Fab. The antibody can be composed of genetically engineered or synthetic fragments of natural antibodies, such as scFv (single chain Fragment variable) species.

The antibodies can be complexed to native antigen probes or to "pseudo-antigen" probes. Examples of pseudo-antigens include antigens from other species. For example, if human C-reactive protein is to be assayed then the pseudo-antigen may include canine, feline, equine, bovine, ovine, porcine or avian C-reactive protein. Pseudo-antigens can also be made by modifying the native antigen. For example, if human C-reactive protein is to be assayed, then the pseudo-antigen may include a monomeric form of the native pentamer, or C-reactive protein which has had its amine, carboxyl, hydroxyl, thiol or disulfide groups chemically modified.

### Using the Sensor to Determine the Presence or Absence of an Antigen

The sensor may be used to determine the presence or absence of an antigen in a sample as follows. Referring to Figures 3 and 4, the strip sensor **100** contains a reaction chamber **124** and a detection chamber **132**. Sample is introduced into reaction chamber **124** via port **166** or **168**. The separation between layers **116** and **156** and the surface energy of their internal surfaces is such that the sample will be drawn into reaction chamber **124** by capillary action. Reaction chamber **124** contains antibodies **164** immobilized to an internal face **126** of the reaction chamber **124**. Pseudo-antigen-probe complexes **162** are bound to antibodies **164** such that substantially all the antibody recognition sites for the antigen are blocked by pseudo-antigen-probe **162**. In this embodiment, the probe is an enzyme.

In Figure 4, the antibody is shown as coated only on one face **126** of the reaction chamber **124**, but it may advantageously be coated on more than one face **126** of the reaction chamber **124** or coated onto a separate support (not illustrated) that is contained in the reaction chamber **124**. However, for ease of fabrication it is typically preferred that the antibodies **164** are only coated on one portion of the reaction chamber **124**, or on a single support material. When a separate support is used to immobilize the antibodies **164**, the support is such that it does not enter the detection chamber **132** during the test. This can be achieved by, for example, adhering the support to at least one face **126** of the reaction chamber **124**, or by selecting the size or shape of the support such that it cannot enter through the sample passageway **134** into detection chamber **132**, or by selecting a support of sufficient density such that it remains on the lower face **126** of the reaction chamber 124 when the sample is transferred to the detection chamber **132**.

When sample fills the reaction chamber **124**, the pseudo-antigen-enzyme probe **162** bound to antibody **164** contacts the sample and a small fraction of the pseudo-antigen-probe dissociates from the antibody **164** and into the sample. Sufficient time is then allowed for the dynamic equilibrium between bound and unbound pseudo-antigen-enzyme probe **162** to be established. If antigen is present in the sample, the antigen, which binds more strongly to the antibody **164** than the pseudo-antigen-enzyme probe **162**, eventually displaces the pseudo-antigen-enzyme probe **162**. Thus each antigen that binds to an immobilized antibody **164** will displace one pseudo-antigen-enzyme probe **162** into solution.

The end of the reaction step is a predetermined time after the sample is introduced into the reaction chamber **124**. The predetermined time is set such that there is sufficient time for substantially all of the antigen in the sample to displace pseudo-antigen-enzyme probe **162** to bind to the antibody **164**. Alternatively, the predetermined time can be set such that a known fraction of the antigen displaces the pseudo-antigen-probe **162** to bind to the antibody **164**.

The time that the sample is introduced into the reaction chamber **124** can be indicated by the user, for example, by depressing a button on a meter (not illustrated) connected to the sensor **100**. This action is used to trigger a timing device (not illustrated). In the case of visual detection, no meter device is necessary. In such an embodiment, the user manually times the reaction period.

In the case where electrochemical detection is used to detect the result of the immuno-reactions, the indication that sample has been introduced into the reaction chamber **124** can be automated. As described above, when sample fills the reaction chamber **124**, a small portion of the detection chamber **132** at its opening **140** into the reaction chamber **124** will be wet by sample. If electrochemical detection is employed then at least two electrodes (not illustrated) are present in the detection chamber **132.** If these electrodes (not illustrated) are placed in the detection chamber **134,** such that at least a portion of each electrode (not illustrated) is contacted by the sample during the filling of the reaction chamber **124**, the presence of the sample will bridge the electrodes (not illustrated) and create an electrical signal which can be used to trigger the timing device.

A predetermined time after the timing device has been triggered, either by the user or automatically, the immuno-reaction phase of the test is deemed to be completed. When the immuno-reaction phase of the test is completed, the vent **158** to the atmosphere is opened. For example, a solenoid activated needle in the meter may be used to pierce layer **156** and/or layer **116,** or additionally layers **114** and **44**, thus opening the distal end **152** of the detection chamber **132** to the atmosphere. The piercing can be automatically performed by the meter, as in the example above, or manually by the user in the case of visual detection wherein no meter may be used, e.g., the user inserts a needle through the layers **156, 116, 114,** and/or **144** into the detection chamber, thereby forming the vent **158.**

The opening of the vent **158** to the atmosphere allows the air trapped in the detection chamber **132** to escape, thereby allowing the detection chamber **132** to be filled with reacted sample from the reaction chamber **124**. The reacted sample will be drawn into the detection chamber **132** due to increased capillary force in the detection chamber **132** compared to that present in the reaction chamber **124**. In a preferred embodiment, the increased capillary force is provided by suitably coating the surfaces **138** and **160** of the detection chamber **132** or, more preferably, by choosing the capillary distance for the detection chamber **132** to be smaller than that of the reaction chamber **124.** In this embodiment, the capillary distance is defined to be the smallest dimension of the chamber.

When the detection chamber **132** is filled, the reagents **172** dissolve into the sample. The enzyme component of the reagent layer **172** reacts with the enzyme substrate and the mediator to produce reduced mediator. This reduced mediator is electrochemically oxidized at an electrode (not illustrated) acting as an anode in the detection chamber **134** to produce an electrical current. In one embodiment, the rate of change of this current with time is used as an indicator of the presence and amount of enzyme that is present in the reacted sample. If the rate of change of current is less than a predetermined threshold value (taking into account that some pseudo-antigen-enzyme probe **162** is liberated into solution as a result of the dynamic equilibrium that is established between the free and bound pseudo-antigen-enzyme probe **162**), then it is indicative of no significant amount of pseudo-antigen-enzyme probe **162** present in the reacted sample, indicating the lack of antigen present in the original sample. If the rate of change of current is higher than the threshold rate, it indicates that pseudo-antigen-enzyme probe **162** is present in the reacted sample in an amount greater than the threshold value, and thus antigen is also present in the sample initially. In one embodiment, the rate of change of the current is used to give a measure of the relative amount of antigen initially present in the sample.

## Claims

1. A disposable device for use in detecting a target antigen in a fluid sample, the device comprising a reaction chamber; an immobilized antibody fixed with the reaction chamber; a reporter complex comprising a probe and a reporter complex antigen, wherein the reporter complex antigen is a target antigen, a pseudo-antigen or a modified-antigen, wherein the probe is linked to the reporter complex antigen, wherein the reporter complex antigen is bound to the immobilized antibody, and wherein the reporter complex antigen binds less strongly than the target antigen to the immobilized antibody; a detection chamber; a sample ingress to the reaction chamber; and a sample passageway between the reaction chamber and the detection chamber.

2. The device of claim 1, wherein the probe is a radioisotope, chromophore, fluorophore or enzyme.

3. The device of claim 2, further comprising an enzyme substrate.

4. The device of claim 3, wherein the enzyme substrate is an oxidizable substrate.

5. The device of claim 3 or 4, wherein the enzyme substrate is supported on a detection chamber interior surface.

6. The device of any one of claims 2 to 5, wherein the enzyme comprises a glucose dehydrogenase.

7. The device of claim 6 when dependent on any one of claims 3 to 5, wherein the enzyme substrate comprises glucose.

8. The device of claim 3 or any claim dependent thereon, further comprising a mediator.

9. The device of claim 8, wherein the mediator is dichlorophenolindophenol, a complex between a transition metal and a nitrogen-containing heteroatomic species or ferricyanide.

10. The device of any one of claims 1 to 9, wherein the sample has a pH, and wherein the device further comprises a buffer that adjusts the pH of the sample.

11. The device of claim 10, wherein the buffer comprises a phosphate or a mellitate.

12. The device of any one of claims 1 to 11, further comprising a stabilizer, wherein the stabilizer stabilizes at least one of the target antigen, the reporter complex antigen, the enzyme or the immobilized antibody.

13. The device of any one of claims 1 to 12, wherein the immobilized antibody is supported on a reaction chamber interior surface.

14. The device of any one of claims 1 to 13, further comprising a support material.

15. The device of claim 14, wherein the support material is contained with the detection chamber, and wherein:
either an enzyme substrate, a mediator or a buffer;
or the immobilized antibody, the reporter complex or an agent that prevents nonspecific binding of proteins to a reaction chamber internal surface
is supported on or contained within the support material.

16. The device of claim 15, wherein the support material comprises a mesh material, a fibrous filling material, a porous material, a sintered powder, a macroporous membrane or a bead.

17. The device of any one of claims 1 to 16, wherein the detection chamber comprises a first electrode and a second electrode.

18. The device of claim 17, wherein at least one of the first electrode and the second electrode comprises aluminium, copper, nickel, chromium, steel, stainless steel, palladium, platinum, gold, iridium carbon, carbon mixed with binder, indium oxide, tin oxide, a conducting polymer or a mixture thereof.

19. The device of any one of claims 1 to 18, wherein a detection chamber wall is transparent to a radiation emitted or absorbed by the probe, wherein the radiation is indicative of the presence or absence of the reporter complex in the detection chamber.

20. The device of any one of claims 1 to 19, further comprising a detector that detects a condition wherein the reaction chamber is substantially filled.

21. The device of any one of claims 1 to 20, wherein the target antigen comprises a human C-reactive protein.

22. The device of any one of claims 1 to 21, wherein the reporter complex antigen is a monomeric C-reactive protein, a C-reactive protein derived from a non-human species or a chemically-modified C-reactive protein wherein an affinity of the chemically-modified C-reactive protein to the antibody is less than an affinity of the human C-reactive protein to the antibody.

23. The device of any one of claims 1 to 22, wherein a wall of the detection chamber or a wall of the reaction chamber further comprises a filler.

24. The device of claim 23, wherein the filler is titanium dioxide, carbon, silica, glass or a mixture thereof.

25. The device of any one of claims 1 to 24, wherein the probe comprises an enzyme co-factor.

26. The device of claim 25, wherein the enzyme co-factor is flavin mononucleotide, flavin adenine dinucleotide, nicotinamide adenine dinucleotide or pyrroloquinoline quinone.

27. The device of claim 25 or claim 26, wherein the enzyme co-factor in linked to the reporter complex antigen through a flexible spacer.

28. The device of any one of claims 25 to 27, further comprising an apoenzyme.

29. The device of any one of claims 1 to 24, wherein the probe comprises an enzyme activity regulator.

30. The device of claim 29, wherein the enzyme activity regulator comprises a kinase or phosphorylase.

31. The device of claim 29 or claim 30, further comprising an enzyme.

32. The device of any one of claims 1 to 24, wherein the probe comprises a protein subunit of a multi-subunit enzyme.

33. A method for determining an amount of a target antigen in a fluid sample, the method comprising the steps of:
placing the fluid sample in a reaction chamber containing an immobilized antibody and a reporter complex comprising a probe linked to a reporter complex antigen, wherein the antibody is fixed within the reaction chamber, wherein the reporter complex antigen is bound to the immobilized antibody, and wherein the reporter complex antigen binds less strongly than the target antigen to the immobilized antibody;
dissociating a portion of the reporter complex antigen from the immobilized antibody into the fluid sample;
binding a portion of the target antigen to the immobilized antibody;
transferring the fluid sample to a detection chamber; and
determining an amount of reporter complex in the fluid sample, wherein the amount of reporter complex is indicative of the amount of target antigen initially in the fluid sample.

34. The method of claim 33, wherein the step of transferring the fluid sample to a detection chamber comprises transferring the fluid sample to an electrochemical cell, the electrochemical cell comprising a first electrode and a second electrode.

35. The method of claim 34, wherein the step of determining an amount of reporter complex in the fluid sample comprises:
applying a potential between the first electrode and the second electrode; and
measuring a current, wherein the current is indicative of an amount of reporter complex present in the fluid sample, and wherein the amount of reporter complex is indicative of the amount of target antigen initially in the fluid sample.

36. The method of any one of claims 33 to 35 wherein the step of transferring the fluid sample to a detection chamber comprises transferring the fluid sample to a detection chamber comprising an electromagnetic radiation transmissive portion.

37. The method of claim 36, wherein the step of determining an amount of reporter complex in the fluid sample comprises the steps of:
exposing the electromagnetic radiation transmissive portion to electromagnetic radiation, whereby the electromagnetic radiation passes through the fluid sample or reflects from the fluid sample; and
monitoring a property of the electromagnetic radiation after it passes through the fluid sample or reflects from the fluid sample, wherein the property is indicative of an amount of reporter complex present in the fluid sample, and wherein the amount of reporter complex is indicative of the amount of target antigen initially in the fluid sample.

## Patentansprüche

1. Wegwerfvorrichtung zur Verwendung beim Nachweis eines Zielantigens in einer Flüssigkeitsprobe, wobei die Vorrichtung eine Reaktionskammer; einen immobilisierten Antikörper, der in der Reaktionskammer fixiert ist; einen Reporterkomplex, der eine Sonde und ein Reporterkomplex-Antigen umfaßt, wobei das Reporterkomplex-Antigen ein Zielantigen, ein Pseudoantigen oder ein modifiziertes Antigen ist, wobei die Sonde mit dem Reporterkomplex-Antigen verknüpft ist, wobei das Reporterkomplex-Antigen an den immobilisierten Antikörper gebunden ist und wobei das Reporterkomplex-Antigen sich weniger stark als das Zielantigen an den immobilisierten Antikörper bindet; eine Nachweiskammer; einen Probeneinlaß zur Reaktionskammer; und einen Probendurchlaß zwischen der Reaktionskammer und der Nachweiskammer umfaßt.

2. Vorrichtung nach Anspruch 1, **dadurch gekennzeichnet, daß** die Sonde ein Radioisotop, Chromophor, Fluorophor oder Enzym ist.

3. Vorrichtung nach Anspruch 2, die weiter ein Enzymsubstrat umfaßt.

4. Vorrichtung nach Anspruch 3, **dadurch gekennzeichnet, daß** das Enzymsubstrat ein oxidierbares Substrat ist.

5. Vorrichtung nach Anspruch 3 oder 4, **dadurch gekennzeichnet, daß** das Enzymsubstrat auf einer Nachweiskammer-Innenfläche geträgert ist.

6. Vorrichtung nach einem der Ansprüche 2 bis 5, **dadurch gekennzeichnet, daß** das Enzym eine Glucose-Dehydrogenase umfaßt.

7. Vorrichtung nach Anspruch 6, wenn abhängig von einem der Ansprüche 3 bis 5, **dadurch gekennzeichnet, daß** das Enzymsubstrat Glucose umfaßt.

8. Vorrichtung nach Anspruch 3 oder irgendeinem davon abhängigen Anspruch, die weiter einen Mediator umfaßt.

9. Vorrichtung nach Anspruch 8, **dadurch gekennzeichnet, daß** der Mediator Dichlorphenolindophenol, ein Komplex zwischen einem Übergangsmetall und einer stickstoffhaltigen Heteroatom-Spezies oder Hexacyanoferrat ist.

10. Vorrichtung nach einem der Ansprüche 1 bis 9, **dadurch gekennzeichnet, daß** die Probe einen pH besitzt und daß die Vorrichtung weiter einen Puffer umfaßt, der den pH der Probe einstellt.

11. Vorrichtung nach Anspruch 10, **dadurch gekennzeichnet, daß** der Puffer ein Phosphat oder ein Mellitat umfaßt.

12. Vorrichtung nach einem der Ansprüche 1 bis 11, die weiter einen Stabilisator umfaßt, wobei der Stabilisator das Zielantigen, das Reporterkomplex-Antigen, das Enzym und/oder den immobilisierten Antikörper stabilisiert.

13. Vorrichtung nach einem der Ansprüche 1 bis 12, **dadurch gekennzeichnet, daß** der immobilisierte Antikörper auf einer Reaktionskammer-Innenfläche geträgert ist.

14. Vorrichtung nach einem der Ansprüche 1 bis 13, die weiter ein Trägermaterial umfaßt.

15. Vorrichtung nach Anspruch 14, **dadurch gekennzeichnet, daß** das Trägermaterial in der Nachweiskammer enthalten ist und daß:
entweder ein Enzymsubstrat, ein Mediator oder ein Puffer;
oder der immobilisierte Antikörper, der Reporterkomplex oder ein Agens, das nichtspezifische Bindung von Proteinen an die Reaktionskammer-Innenfläche verhindert,
auf dem Trägermaterial geträgert oder in diesem enthalten ist.

16. Vorrichtung nach Anspruch 15, **dadurch gekennzeichnet, daß** das Trägermaterial ein Netzmaterial, ein faseriges Füllmaterial, ein poröses Material, ein gesintertes Pulver, eine makroporöse Membran oder eine Perle umfaßt.

17. Vorrichtung nach einem der Ansprüche 1 bis 16, **dadurch gekennzeichnet, daß** die Nachweiskammer eine erste Elektrode und eine zweite Elektrode umfaßt.

18. Vorrichtung nach Anspruch 17, **dadurch gekennzeichnet, daß** die erste Elektrode und/oder die zweite Elektrode Aluminium, Kupfer, Nickel, Chrom, Stahl, rostfreien Stahl, Palladium, Platin, Gold, Iridiumkohlenstoff, Kohlenstoff, vermischt mit Bindemittel, Indiumoxid, Zinnoxid, ein leitendes Polymer oder eine Mischung davon umfaßt.

19. Vorrichtung nach einem der Ansprüche 1 bis 18, **dadurch gekennzeichnet, daß** eine Nachweiskammerwand für eine von der Sonde abgegebene oder absorbierte Strahlung durchlässig ist, wobei die Strahlung das Vorhandensein oder Nichtvorhandensein des Reporterkomplexes in der Nachweiskammer anzeigt.

20. Vorrichtung nach einem der Ansprüche 1 bis 19, die weiter einen Detektor umfaßt, der einen Zustand nachweist, in dem die Reaktionskammer im wesentlichen gefüllt ist.

21. Vorrichtung nach einem der Ansprüche 1 bis 20, **dadurch gekennzeichnet, daß** das Zielantigen ein menschliches C-reaktives Protein umfaßt.

22. Vorrichtung nach einem der Ansprüche 1 bis 21, **dadurch gekennzeichnet, daß** das Reporterkomplex-Antigen ein monomeres C-reaktives Protein, ein C-reaktives Protein, das aus einer nicht-menschlichen Spezies gewonnen ist, oder ein chemisch modifiziertes C-reaktives Protein ist, wobei eine Affinität des chemisch modifizierten C-reaktiven Proteins zum Antikörper geringer ist als eine Affinität des menschlichen C-reaktiven Proteins zum Antikörper.

23. Vorrichtung nach einem der Ansprüche 1 bis 22, **dadurch gekennzeichnet, daß** eine Wand der Nachweiskammer oder eine Wand der Reaktionskammer weiter einen Füllstoff umfaßt.

24. Vorrichtung nach Anspruch 23, **dadurch gekennzeichnet, daß** der Füllstoff Titandioxid, Kohlenstoff, Siliciumdioxid, Glas oder eine Mischung davon ist.

25. Vorrichtung nach einem der Ansprüche 1 bis 24, **dadurch gekennzeichnet, daß** die Sonde einen Enzym-Cofaktor umfaßt.

26. Vorrichtung nach Anspruch 25, **dadurch gekennzeichnet, daß** der Enzym-Cofaktor Flavin-Mononukleotid, Flavin-Adenin-Dinukleotid, Nikotinamid-Adenin-Dinukleotid oder Pyrrolochinolinchinon ist.

27. Vorrichtung nach Anspruch 25 oder Anspruch 26, **dadurch gekennzeichnet, daß** der Enzym-Cofaktor mit dem Reporterkomplex-Antigen durch einen flexiblen Spacer verknüpft ist.

28. Vorrichtung nach einem der Ansprüche 25 bis 27, die weiter ein Apoenzym umfaßt.

29. Vorrichtung nach einem der Ansprüche 1 bis 24, **dadurch gekennzeichnet, daß** die Sonde einen Enzymaktivitätsregulator umfaßt.

30. Vorrichtung nach Anspruch 29, **dadurch gekennzeichnet, daß** der Enzymaktivitätsregulator eine Kinase oder Phosphorylase umfaßt.

31. Vorrichtung nach Anspruch 29 oder Anspruch 30, die weiter ein Enyzm umfaßt.

32. Vorrichtung nach einem der Ansprüche 1 bis 24, **dadurch gekennzeichnet, daß** die Sonde eine Protein-Untereinheit eines Enzyms mit mehreren Untereinheiten umfaßt.

33. Verfahren zur Bestimmung einer Menge eines Zielantigens in einer Flüssigkeitsprobe, wobei das Verfahren die Schritte umfaßt:
Einbringen der Flüssigkeitsprobe in eine Reaktionskammer, die einen immobilisierten Antikörper und einen Reporterkomplex umfaßt, der eine Sonde umfaßt, die an ein Reporterkomplex-Antigen gebunden ist, wobei der Antikörper innerhalb der Reaktionskammer fixiert ist, wobei das Reporterkomplex-Antigen an den immobilisierten Antikörper gebunden ist und wobei das Reporterkomplex-Antigen sich weniger stark als an das Zielantigen an den immobilisierten Antikörper bindet;
Dissoziieren eines Teils des Reporterkomplex-Antigens vom immobilisierten Antikörper in die Flüssigkeitsprobe hinein;
Binden eines Teils des Zielantigens an den immobilisierten Antikörper;
Überführen der Flüssigkeitsprobe in eine Nachweiskammer; und
Bestimmen einer Menge an Reporterkomplex in der Flüssigkeitsprobe, wobei die Menge an Reporterkomplex die Menge an Zielantigen anzeigt, die anfänglich in der Flüssigkeitsprobe vorliegt.

34. Verfahren nach Anspruch 33, **dadurch gekennzeichnet, daß** der Schritt des Überführens der Flüssigkeitsprobe in eine Nachweiskammer das Überführen der Flüssigkeitsprobe in eine elektrochemische Zelle umfaßt, wobei die elektrochemische Zelle eine erste Elektrode und eine zweite Elektrode umfaßt.

35. Verfahren nach Anspruch 34, **dadurch gekennzeichnet, daß** der Schritt des Bestimmens einer Menge an Reporterkomplex in der Flüssigkeitsprobe umfaßt:
Anlegen eines Potentials zwischen der ersten Elektrode und der zweiten Elektrode; und
Messen eines Stroms, wobei der Strom eine Menge an Reporterkomplex anzeigt, die in der Flüssigkeitsprobe vorliegt, und wobei die Menge an Reporterkomplex die Menge an Zielantigen anzeigt, die ursprünglich in der Flüssigkeitsprobe vorliegt.

36. Verfahren nach einem der Ansprüche 33 bis 35, **dadurch gekennzeichnet, daß** der Schritt des Überführens der Flüssigkeitsprobe in eine Nachweiskammer das Überführen der Flüssigkeitsprobe in eine Nachweiskammer umfaßt, die einen für elektromagnetische Strahlung durchlässigen Abschnitt umfaßt.

37. Verfahren nach Anspruch 36, **dadurch gekennzeichnet, daß** der Schritt des Bestimmens einer Menge an Reporterkomplex in der Flüssigkeitsprobe die Schritte umfaßt:
Aussetzen des für elektromagnetische Strahlung durchlässigen Abschnitts gegenüber elektromagnetischer Strahlung, wodurch die elektromagnetische Strahlung durch die Flüssigkeitsprobe hindurchgeht oder von der Flüssigkeitsprobe reflektiert; und
Überwachen einer Eigenschaft der elektromagnetischen Strahlung, nachdem sie durch die Flüssigkeitsprobe hindurchgegangen ist oder von der Flüssigkeitsprobe reflektiert ist, wobei die Eigenschaft eine Menge an Reporterkomplex anzeigt, die in der Flüssigkeitsprobe vorliegt, und wobei die Menge an Reporterkomplex die Menge an Zielantigen anzeigt, die ursprünglich in der Flüssigkeitsprobe vorliegt.

## Revendications

1. Dispositif jetable destiné à la détection d'un antigène cible dans un échantillon de fluide, lequel dispositif comprend : une chambre de réaction ; un anticorps immobilisé fixé à la chambre de réaction ; un complexe rapporteur comprenant une sonde et un antigène de complexe rapporteur, l'antigène de complexe rapporteur consistant en un antigène cible, un pseudo-antigène ou un antigène modifié, la sonde étant liée à l'antigène de complexe rapporteur, l'antigène de complexe rapporteur étant lié à l'anticorps immobilisé, et l'antigène de complexe rapporteur se liant moins fortement que l'antigène cible à l'anticorps immobilisé ; une chambre de détection ; une admission d'échantillon vers la chambre de réaction ; et un passage pour échantillon entre la chambre de réaction et la chambre de détection.

2. Dispositif selon la revendication 1, dans lequel la sonde consiste en un radio-isotope, en un chromophore, en un fluorophore ou en une enzyme.

3. Dispositif selon la revendication 2, comprenant en outre un substrat pour enzyme.

4. Dispositif selon la revendication 3, dans lequel le substrat pour enzyme consiste en un substrat oxydable.

5. Dispositif selon la revendication 3 ou 4, dans lequel le substrat pour enzyme est supporté sur la surface interne de la chambre de détection.

6. Dispositif selon l'une quelconque des revendications 2 à 5, dans lequel l'enzyme comprend une glucose déshydrogénase.

7. Dispositif selon la revendication 6 lorsqu'elle dépend de l'une quelconque des revendications 3 à 5, dans lequel le substrat pour enzyme comprend du glucose.

8. Dispositif selon la revendication 3 ou une quelconque revendication en dépendant, comprenant en outre un médiateur.

9. Dispositif selon la revendication 8, dans lequel le médiateur consiste en du dichloro-phénolindophénol, un complexe entre un métal de transition et des espèces hétéro-atomiques contenant de l'azote, ou du ferricyanure.

10. Dispositif selon l'une quelconque des revendications 1 à 9, dans lequel l'échantillon possède un certain pH, le dispositif comprenant en outre un tampon qui ajuste le pH de l'échantillon.

11. Dispositif selon la revendication 10, dans lequel le tampon comprend un phosphate ou un mellitate.

12. Dispositif selon l'une quelconque des revendications 1 à 11, comprenant en outre un stabilisateur, lequel stabilisateur stabilise l'un au moins des éléments que sont l'antigène cible, l'antigène de complexe rapporteur, l'enzyme et l'anticorps immobilisé.

13. Dispositif selon l'une quelconque des revendications 1 à 12, dans lequel l'anticorps immobilisé est supporté sur une surface interne de la chambre de réaction.

14. Dispositif selon l'une quelconque des revendications 1 à 13, comprenant en outre un matériau de support.

15. Dispositif selon la revendication 14, dans lequel le matériau de support est contenu dans la chambre de réaction, et dans lequel :
> soit un substrat pour enzyme, un médiateur ou un tampon,
➢ soit l'anticorps immobilisé, le complexe rapporteur ou un agent empêchant la liaison non spécifique de protéines à la surface interne de la chambre de réaction
est supporté sur ou contenu dans le matériau de support.

16. Dispositif selon la revendication 15, dans lequel le matériau de support comprend un matériau à maillage, un matériau de remplissage fibreux, un matériau poreux, une poudre frittée, une membrane macro-poreuse ou une perle.

17. Dispositif selon l'une quelconque des revendications 1 à 16, dans lequel la chambre de détection comprend une première électrode et une seconde électrode.

18. Dispositif selon la revendication 17, dans lequel la première électrode et/ou la seconde électrode comprennent de l'aluminium, du cuivre, du nickel, du chrome, de l'acier, de l'acier inoxydable, du palladium, du platine, de l'or, de l'iridium, du carbone, du carbone mélangé à un liant, de l'oxyde d'indium, de l'oxyde d'étain, un polymère conducteur ou un mélange de ces derniers.

19. Dispositif selon l'une quelconque des revendications 1 à 18, dans lequel une paroi de la chambre de détection est transparente aux rayonnements émis ou absorbés par la sonde, lequel rayonnement indique la présence ou l'absence du complexe rapporteur dans la chambre de détection.

20. Dispositif selon l'une quelconque des revendications 1 à 19, comprenant en outre un détecteur qui va détecter un état dans lequel la chambre de réaction est essentiellement remplie.

21. Dispositif selon l'une quelconque des revendications 1 à 20, dans lequel l'antigène cible comprend une protéine C-réactive humaine.

22. Dispositif selon l'une quelconque des revendications 1 à 21, dans lequel l'antigène de complexe rapporteur est une protéine C-réactive monomère, une protéine C-réactive dérivée d'une espèce non humaine ou une protéine C-réactive modifiée chimiquement, l'affinité de la protéine C-réactive modifiée chimiquement envers l'anticorps étant moindre que l'affinité de la protéine C-réactive humaine envers l'anticorps.

23. Dispositif selon l'une quelconque des revendications 1 à 22, dans lequel une paroi de la chambre de détection ou une paroi de la chambre de réaction comprend en outre une matière de charge.

24. Dispositif selon la revendication 23, dans lequel la matière de charge consiste en du dioxyde de titane, du carbone, de la silice, du verre ou un mélange de ces derniers.

25. Dispositif selon l'une quelconque des revendications 1 à 24, dans lequel la sonde comprend un co-facteur d'enzyme.

26. Dispositif selon la revendication 25, dans lequel le co-facteur d'enzyme consiste en un mono-nucléotide de flavine, en un dinucléotide d'adénine de flavine, en un dinucléotide d'adénine de nicotinamide ou en de la quinone de pyrroloquinoline.

27. Dispositif selon la revendication 25 ou 26, dans lequel le co-facteur d'enzyme est lié à l'antigène de complexe rapporteur par un espaceur flexible.

28. Dispositif selon l'une quelconque des revendications 25 à 27, comprenant en outre une apoenzyme.

29. Dispositif selon l'une quelconque des revendications 1 à 24, dans lequel la sonde comprend un régulateur d'activité d'enzyme.

30. Dispositif selon la revendication 29, dans lequel le régulateur d'activité d'enzyme comprend une kinase ou une phosphorylase.

31. Dispositif selon la revendication 29 ou 30, comprenant en outre une enzyme.

32. Dispositif selon l'une quelconque des revendications 1 à 24, dans lequel la sonde comprend une sous-unité de protéine d'une enzyme à sous-unités multiples.

33. Procédé permettant de déterminer une quantité d'un antigène cible dans un échantillon de fluide, lequel procédé comprend les étapes consistant à :
➢ placer l'échantillon de fluide dans une chambre de réaction contenant un anticorps immobilisé et un complexe rapporteur comprenant une sonde liée à un antigène de complexe rapporteur, l'anticorps étant fixé dans la chambre de réaction, l'antigène de complexe rapporteur étant lié à l'anticorps immobilisé, et l'antigène de complexe rapporteur se liant moins fortement que l'antigène cible à l'anticorps immobilisé ;
➢ dissocier une partie de l'antigène de complexe rapporteur de l'anticorps immobilisé dans l'échantillon de fluide ;
➢ lier une partie de l'antigène cible à l'anticorps immobilisé ;
➢ transférer l'échantillon de fluide vers une chambre de détection ; et
➢ déterminer une quantité de complexe rapporteur dans l'échantillon de fluide, la quantité de complexe rapporteur indiquant la quantité d'antigène cible se trouvant initialement dans l'échantillon de fluide.

34. Procédé selon la revendication 33, dans lequel l'étape de transfert de l'échantillon de fluide vers une chambre de détection consiste à transférer l'échantillon de fluide vers une cellule électrochimique, laquelle cellule électrochimique comprend une première électrode et une seconde électrode.

35. Procédé selon la revendication 34, dans lequel l'étape consistant à déterminer une quantité de complexe rapporteur dans l'échantillon de fluide consiste à :
➢ appliquer un potentiel entre la première élec-trode et la seconde électrode ; et
➢ mesurer un courant, le courant indiquant la quantité de complexe rapporteur présent dans l'échantillon de fluide, et la quantité de complexe rapporteur indiquant la quantité d'antigène cible initialement présente dans l'échantillon de fluide.

36. Procédé selon l'une quelconque des revendications 33 à 35, dans lequel l'étape consistant à transférer l'échantillon de fluide vers une chambre de détection consiste à transférer l'échantillon de fluide vers une chambre de détection comprenant une partie transmettant le rayonnement électromagnétique.

37. Procédé selon la revendication 36, dans lequel l'étape consistant à déterminer une quantité de complexe rapporteur dans l'échantillon de fluide comprend les étapes consistant à :
➢ exposer la partie transmettant le rayonnement électromagnétique à un rayonnement électromagnétique, le rayonnement électromagnétique passant à travers l'échantillon de fluide ou se réfléchissant depuis l'échantillon de fluide ; et
➢ contrôler une propriété du rayonnement électromagnétique après son passage à travers l'échantillon de fluide ou sa réflexion depuis l'échantillon de fluide, cette propriété indiquant une quantité de complexe rapporteur présent dans l'échantillon de fluide, et la quantité de complexe rapporteur indiquant la quantité d'antigène cible se trouvant initialement dans l'échantillon de fluide.
